(19) Europäisches Patentamt
European Patent Office
Office européen des brevets

(11) Numéro de publication : **0 189 698**
**B1**

(12) # FASCICULE DE BREVET EUROPÉEN

(45) Date de publication du fascicule du brevet : **30.09.87**

(51) Int. Cl.⁴ : **C 08 F 2/38**, A 62 D 1/00

(21) Numéro de dépôt : **85402546.7**

(22) Date de dépôt : **18.12.85**

(54) **Télomères fluorés à groupements hydrophiles, leur procédé de préparation et leur utilisation comme agents tensioactifs en milieu aqueux.**

(30) Priorité : **26.12.84 FR 8419834**

(43) Date de publication de la demande : **06.08.86 Bulletin 86/32**

(45) Mention de la délivrance du brevet : **30.09.87 Bulletin 87/40**

(84) Etats contractants désignés : **AT BE CH DE FR GB IT LI LU NL SE**

(56) Documents cités : **EP-A- 0 019 584**

(73) Titulaire : **ATOCHEM**
**4 & 8, Cours Michelet La Défense 10**
**F-92800 Puteaux (FR)**

(72) Inventeur : **Boutevin, Bernard**
**Les Terres Blanches 1 rue Anselme Mathieu**
**F-34000 Montpellier (FR)**
Inventeur : **Pietrasanta, Yves**
**14, Place Merli Pougade**
**F-34140 Meze (FR)**
Inventeur : **Taha, Mohamed**
**Cité 160 Logements Bat. E 13**
**27000 Mostaganem (DZ)**
Inventeur : **Lantz, André**
**Domaine de la Hêtraie**
**F-69390 Vernaison (FR)**

(74) Mandataire : **Leboulenger, Jean et al**
**ATOCHEM Département Propriété Industrielle**
**F-92091 Paris la Défense 10 Cedex 42 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 189 698 B1

**Description**

La présente invention concerne des composés biséquencés comportant une séquence totalement ou partiellement fluorée et une séquence hydrocarbonée hydrophile, utilisables notamment comme agents tensioactifs, en particulier dans le domaine des émulseurs anti-incendie.

Les composés perfluorés sont bien connus pour leur aptitude à abaisser considérablement la tension superficielle des liquides dans lesquels ils sont dissous, même à de très faibles concentrations. Cependant, pour pouvoir être utilisés comme agents tensioactifs en milieu aqueux, ils doivent être rendus hydrosolubles. A cet effet, on adjoint à la chaîne fluorée un groupement ou une séquence hydrophile qui peut être de caractère anionique, cationique, amphotère ou non ionique. De tels agents tensioactifs sont bien connus (voir, par exemple, R. E. Banks, « Organofluorine chemicals and their industrial applications » — Ellis Horwood Ltd, London 1979, pages 214-234).

Dans la plupart des tensioactifs fluorés, la partie hydrophile est constituée par un groupement chimique classique tel qu'un groupe carboxylate, sulfonate, ammonium quaternaire, bétaïne, etc..., mais on connaît aussi des tensioactifs fluorés dans lesquels la partie hydrophile est un enchaînement oligomère hydrophile. Dans le brevet U.S. 2 723 999 sont ainsi décrits des produits répondant à la formule :

$$C_xF_{2x+1}CH_2O(C_2H_4O)_yH \qquad \text{(avec } y = 1 \text{ à } 200)$$

et dans le brevet U.S. 4 377 710 des produits de formule :

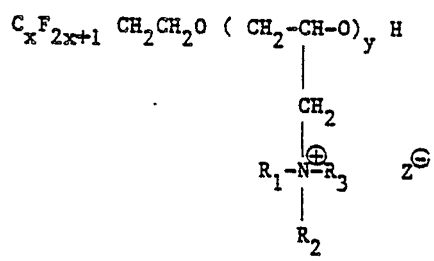

(avec y = 1 à 20)

L'avantage essentiel de ce type de tensioactifs provient de la possibilité de faire varier facilement leur balance de l'hydrophobie et de l'hydrophilie. Le brevet européen 19 584 décrit par ailleurs la préparation de produits du type :

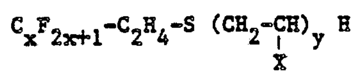

où y peut varier de 4 à 500 et X est notamment un groupe COOH ou $CONH_2$, par télomérisation radicalaire d'un thiol $C_xF_{2x+1}C_2H_4SH$ avec un monomère vinylique tel que, par exemple, l'acide acrylique ou l'acrylamide.

La réaction des iodures de perfluoroalkyle avec des oléfines

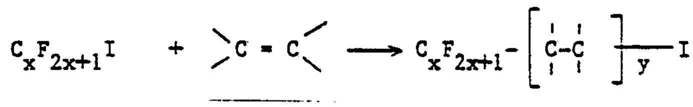

a par ailleurs été déjà très étudiée, en particulier par N. O. Brace (brevet US 3 145 222 ainsi que J. Org. Chem. 1962, 27, 3033 — 1962, 27, 4491 et 1967, 32, 430). Cependant, dans la plupart des cas, cette réaction ne fournit qu'un produit de monoaddition (y = 1) et seules certaines oléfines très réactives donnent des oligomères où y est supérieur à 1 ; ainsi l'acrylate d'éthyle fournit un produit avec y = 8 et le styrène un dérivé avec y = 2. Si la réaction radicalaire des iodures de perfluoroalkyle avec des oléfines est connue, la réaction similaire des dérivés du type $C_xF_{2x+1}$—$CH_2I$ ou $C_xF_{2x+1}$—$C_2H_4I$ ne l'est par contre pas.

Il a maintenant été trouvé qu'en faisant réagir les télogènes fluorés avec certaines oléfines (par exemple, acrylamide ou acide acrylique), on peut obtenir des télomères fluorés dans lesquels le degré de télomérisation moyen peut varier dans de larges limites.

La présente invention a donc pour objet de nouveaux composés biséquencés constitués par des télomères fluorés qu'on peut représenter par la formule générale :

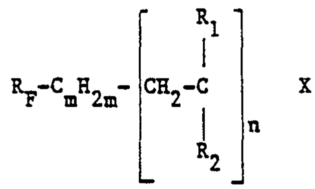 (I)

obtenus par télomérisation radicalaire d'un monomère de formule :

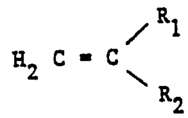 (II)

avec un télogène fluoré de formule :

$$R_F—C_mH_{2m}—X \qquad (III)$$

Dans les formules (I) à (III), $R_F$ représente un radical poly- ou perfluoré linéaire ou ramifié, X représente un atome d'iode, de brome ou de chlore, m est égal à 0, 1 ou 2, n est un nombre allant de 5 à 1 000, $R_1$ représente un atome d'hydrogène ou un radical méthyle et $R_2$ représente un groupe —COOH ou —$CONR_3R_4$, les symboles $R_3$ et $R_4$, identiques ou différents, représentant chacun un atome d'hydrogène ou un radical alkyle ou hydroxyalkyle.

Comme exemples de radicaux $R_F$, on peut citer plus particulièrement les radicaux perfluoroalkyle, linéaires ou ramifiés, de formule :

$$C_xF_{2x+1}—.$$

où x est un nombre entier allant de 1 à 20 et, de préférence, compris entre 4 et 16.

Comme télogènes fluorés (III), on préfère utiliser ceux dans lesquels X est un atome d'iode et m est égal à 0 ou 2.

Comme monomères de formule (II), on peut citer plus particulièrement l'acrylamide, la méthacrylamide, la N-isopropyl-acrylamide, la N-hydroxyméthyl-acrylamide, l'acide acrylique et l'acide méthacrylique.

Les télogènes fluorés de formule (III) sont des produits connus. Ceux de formules $R_F$—I et $R_F$—$C_2H_4$—I, préférentiellement utilisés selon l'invention, ont par exemple été décrits par R. N. Haszeldine (J.C.S. 1949, 2856-2861 et J.C.S. 1953, 3761). Les dérivés $R_FC_2H_4I$ sont en général obtenus par réaction radicalaire des $R_FI$ avec l'éthylène :

$$R_FI + C_2H_4 \longrightarrow R_FC_2H_4I$$

cette réaction conduisant presque exclusivement au produit de mono-addition $R_FC_2H_4I$ et ne fournissant pas ou très peu de télomères supérieurs tels que $R_F(C_2H_4)_2I$ ou $R_F(C_2H_4)_3I$. Pour la préparation des télogènes $R_FCH_2I$, on pourra se reporter à l'article de G. V. D. Tiers dans J.A.C.S. 1953, 75, 5978, ainsi qu'à Phosphorus and Sulfur 1984, 20, 197.

Les proportions de monomère (II) et de télogène (III) à mettre en œuvre peuvent varier dans de larges limites et sont essentiellement fonction du degré de télomérisation moyen en nombre DPn désiré, c'est-à-dire de la valeur de n, qui est relié d'une part à la constante de transfert du télogène ($C_T$) et d'autre part au rapport molaire : télogène/monomère (R) par la relation :

$$1/\overline{DPn} = 1/\overline{DPo} + C_T \cdot R$$

$\overline{DPo}$ représentant le degré de polymérisation en l'absence de transfert de chaîne. Pour un télogène donné, la constante de transfert $C_T$ peut être déterminée de façon connue en soi (voir exemples 1 à 9 ci-après) et correspond à la pente de la courbe représentative, en fonction de R, de l'inverse des $\overline{DPn}$ (obtenus au début d'opérations réalisées en faisant varier le rapport R et déterminés par analyse centésimale du fluor des télomères purifiés).

Selon les télomères qu'on désire obtenir, on peut opérer en discontinu, en semi-continu ou en continu.

Pour amorcer la réaction, on utilise un composé se décomposant sous l'effet de la chaleur en deux radicaux. Ce composé peut être par exemple un peroxyde comme le peroxyde de tert.butyle et le peroxyde de benzoyle, un hydroperoxyde comme l'hydroperoxyde de cumène et l'hydroperoxyde de tert.butyle, un composé azoïque comme l'azobisisobutyronitrile, un percarbonate comme le perbenzoate de tert.butyle. On peut également amorcer la réaction par rayonnement UV en présence ou non de photo-initiateurs tels que, par exemple, les éthers de benzoïne, la benzophénone, la méthyl-2 anthraquinone et le benzile. La quantité d'amorceur ou de photo-initiateur à utiliser peut varier entre 0,1 et 5 % par rapport

au poids de monomère (II). Lorsqu'on met en œuvre un télogène fluoré (III) où m est égal à zéro, on opère de préférence à l'abri de la lumière.

La réaction peut être réalisée dans un solvant inerte tel que, par exemple, un alcool, un nitrile, le tétrahydrofuranne, une cétone, le diméthylformamide. On opère généralement à une température comprise entre 30 et 100 °C.

Les télomères fluorés obtenus selon le procédé de l'invention sont d'excellents agents tensioactifs, en particulier en milieu aqueux. Par rapport aux autres tensioactifs fluorés biséquencés connus où la partie hydrophile de la molécule est une chaîne oligomère, par exemple les produits cités ci-dessus, ils présentent l'avantage essentiel d'être obtenus à partir de télogènes facilement accessibles, par exemple les $R_FI$ et $R_FC_2H_4I$. Les produits similaires décrits dans les brevets US 2 723 999, US 4 377 710 et EP 19584 précités sont en effet obtenus à partir de produits tels que $R_FC_2H_4OH$ ou $R_FC_2H_4SH$ qui sont eux-mêmes fabriqués à partir des $R_FC_2H_4I$ et donc des $R_FI$. Le procédé de l'invention qui permet d'utiliser directement les $R_FI$ et les $R_FC_2H_4I$ est ainsi économiquement beaucoup plus intéressant.

Les nouveaux télomères fluorés selon l'invention peuvent être utilisés dans de nombreux domaines, en particulier dans tous les cas où l'on cherche à abaisser la tension superficielle d'un liquide ou à conférer à un substrat un effet aussi bien hydrophobe qu'oléophobe. Ils peuvent par exemple être utilisés comme mouillants, agents moussants, émulsifiants, dispersants, agents d'étalement de produits tels que cires, vernis, peintures ou encres, et additifs aux lubrifiants.

Une application particulièrement intéressante des télomères selon l'invention concerne la préparation d'émulseurs anti-incendie fluoro-protéiniques. L'utilisation d'émulseurs protéiniques dans le domaine de l'extinction est très répandue (voir par exemple « Agents extincteurs » — Centre National de Prévention et de Protection, PARIS 1982). Les émulseurs à base protéinique sont des hydrolysats de protéines animales ou végétales, les matières premières étant surtout des poudres de cornes et sabots bovins, des plumes broyées, du sang... L'hydrolyse de ces matières protéiniques est principalement réalisée à l'aide de soude ou de chaux et ces émulseurs protéiniques sont très utilisés pour la protection des gros risques d'incendie rencontré dans l'industrie pétrolière (raffineries, dépôts d'hydrocarbures, navires pétroliers...). Les émulseurs protéiniques permettent d'obtenir une mousse très étanche, résistant remarquablement au feu, et à décantation lente. Les mousses obtenues à partir des émulseurs protéiniques présentent néanmoins certains inconvénients, à savoir :

la mousse est très compacte et s'étale difficilement sur le foyer,

la résistance de la mousse à la contamination par les hydrocarbures est mauvaise, ce qui interdit par exemple son utilisation en projection violente.

Pour améliorer l'efficacité de ces émulseurs protéiniques, il est connu de leur incorporer de très faibles quantités de tensioactifs fluorés (voir par exemple les brevets US 3 475 333, FR 2 007 254, FR 2 010 842 et EP 19584). Ce dopage des émulseurs protéiniques à l'aide de tensioactifs fluorés permet d'obtenir une mousse plus fluide, moins compacte et ayant une meilleure résistance à la contamination par les hydrocarbures. La plupart des tensioactifs fluorés permettent d'améliorer la fluidité de la mousse c'est-à-dire sa facilité d'étalement, mais en général les autres propriétés de la mousse sont moins bonnes ; le pouvoir moussant ou le foisonnement de la mousse (rapport entre le volume de mousse produit et le volume de solution moussante ayant servi à produire cette mousse) est en général moins élevé et la mousse formée est très souvent beaucoup moins stable c'est-à-dire décante plus rapidement pour retourner à l'état de solution moussante (une décantation lente est un facteur de qualité car la mousse assure une couverture stable pendant un long moment). Par ailleurs, la plupart des tensioactifs fluorés ne permettent pas d'améliorer de façon sensible la résistance à la contamination par les hydrocarbures qui est une propriété essentielle d'un bon émulseur.

Il a été trouvé que les tensioactifs fluorés selon l'invention et en particulier les produits obtenus par télomérisation de l'acrylamide avec les $R_FI$ et $R_FC_2H_4I$ permettent, par incorporation dans un émulseur protéinique standard, d'obtenir des émulseurs présentant d'excellentes caractéristiques de mousse :

pouvoir moussant
taux de foisonnement
temps de décantation
fluidité de la mousse
contamination par les hydrocarbures

et donc particulièrement efficaces pour la lutte contre l'incendie. La quantité de tensioactif fluoré selon l'invention à incorporer dans l'émulseur protéinique est en général telle qu'après dilution à l'eau de l'émulseur la solution diluée comprenne de 0,005 à 0,5 % en poids de tensioactif fluoré, des doses inférieures ou supérieures pouvant évidemment être utilisées si on le désire.

Les exemples suivants illustrent l'invention.

Exemples 1 à 9 : Télomérisation de l'acrylamide avec $C_6F_{13}CH_2CH_2I$
(Détermination de la constante de transfert).

On effectue neuf opérations de télomérisation en faisant varier le rapport molaire (Ro) du composé $C_6F_{13}CH_2CH_2I$ à l'acrylamide. La réaction est réalisée à 80 °C en solution dans le butyronitrile (1,5 fois le volume d'acrylamide) et en présence de 2 % d'azobisisobutyronitrile par rapport au poids d'acrylamide.

Elle est interrompue à un avancement « α » très faible (2 %) déterminé par pesée d'un échantillon.

Le précipité blanc obtenu est filtré, lavé plusieurs fois à l'acétone, séché dans une étuve à vide et soumis à l'analyse élémentaire du fluor pour calculer le DPn par la relation :

$$\% F = (13 \times 18{,}99 \times 100)/(71 \times DPn + 473{,}87)$$

Le tableau suivant rassemble les résultats obtenus en fonction du rapport molaire Ro.

Tableau 1

| EXEMPLE | Ro | % F | $\overline{DPn}$ |
|---|---|---|---|
| 1 | 0,075 | 0,40 | 863 |
| 2 | 0,25 | 0,58 | 593 |
| 3 | 0,50 | 0,68 | 505 |
| 4 | 0,75 | 1,08 | 315 |
| 5 | 1 | 1,28 | 265 |
| 6 | 1,5 | 1,75 | 192 |
| 7 | 2 | 2,04 | 163 |
| 8 | 3 | 2,98 | 110 |
| 9 | 6 | 5,49 | 57 |

En portant les valeurs $1/\overline{DPn}$ en fonction de Ro, on obtient une droite dont la pente correspond à la constante de transfert du télogène $C_6F_{13}CH_2CH_2I$ à 80 °C et on trouve $C_{80} = 28 \cdot 10^{-4}$.

En analyse I. R., les télomères obtenus présentent, en plus des bandes d'absorption caractéristiques d'un polyacrylamide, une multitude de bandes entre 1 100 et 1 400 $cm^{-1}$. Ces bandes sont caractéristiques des produits perfluorés et leurs intensités relatives augmentent quand le $\overline{DPn}$ diminue.

Exemples 10 à 16

On opère comme aux exemples 1 à 9, sauf que la réaction n'est interrompue qu'après la disparition totale de l'acrylamide (environ 3 heures).

Les résultats obtenus dans sept opérations distinctes sont rassemblés dans le tableau suivant.

Tableau 2

| EXEMPLE | Ro | % F | $\overline{DPn}$ |
|---|---|---|---|
| 10 | 1 | 1,80 | 186 |
| 11 | 1,5 | 1,65 | 204 |
| 12 | 2 | 2,32 | 143 |
| 13 | 3 | 3,57 | 90 |
| 14 | 4 | 4,19 | 76 |
| 15 | 6 | 6,04 | 51 |
| 16 | 8 | 5,65 | 55 |

Exemple 17

Dans un réacteur en verre on introduit 50 g du composé $C_6F_{13}—CH_2CH_2I$ et 25 ml d'alcool isopropylique. Le mélange est porté à reflux sous agitation et on introduit en 8 heures une solution de 50 g d'acrylamide, 1,4 g d'azobisisobutyronitrile et 200 ml d'alcool isopropylique. Lorsque l'addition est

terminée, le mélange est encore maintenu à reflux pendant une heure, puis refroidi à température ambiante.

Le milieu réactionnel est ensuite filtré et le solide est lavé avec deux fois 20 ml d'isopropanol, puis essoré et séché sous vide. On obtient ainsi 45 g d'un solide pulvérulent blanc dont la teneur en fluor est égale à 2,57 %, ce qui correspond à un $\overline{DPn}$ de 129, c'est-à-dire un produit qu'on peut représenter par la formule :

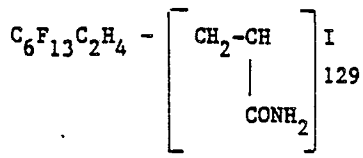

Ce produit est soluble dans l'eau et une solution aqueuse à 1 000 ppm a une tension superficielle de 28,9 mN/m à 25 °C. A 100 ppm, le même produit permet d'obtenir une tension superficielle égale à 44,8 mN/m.

Exemples 18 à 24 : Télomérisation de l'acrylamide avec $C_6F_{13}I$
(Détermination de la constante de transfert)

En opérant comme aux exemples 1 à 9 avec le composé $C_6F_{13}I$, dans un ballon inactinique, on obtient les résultats suivants :

Tableau 3

| EXEMPLE | Ro | % F | $\overline{DPn}$ |
|---|---|---|---|
| 18 | 0,1 | 1,94 | 173 |
| 19 | 0,2 | 3,38 | 96 |
| 20 | 0,3 | 5,35 | 58 |
| 21 | 0,4 | 6,53 | 47 |
| 22 | 0,5 | 8,42 | 35 |
| 23 | 0,7 | 11,04 | 25 |
| 24 | 0,9 | 13,17 | 20 |

La constante de transfert au télogène $C_6F_{13}I$ à 80 °C est de $555 \cdot 10^{-4}$.

Exemples 25 à 34

On opère comme aux exemples 1 à 9, mais on utilise $C_6F_{13}I$ et on n'interrompt la réaction qu'après la disparition complète de l'acrylamide.

Le tableau suivant rassemble les résultats obtenus dans 10 opérations effectuées avec des rapports molaires allant de 0,1 à 4.

Tableau 4

| EXEMPLE | Ro | % F | $\overline{DPn}$ |
|---|---|---|---|
| 25 | 0,1 | 2,53 | 131 |
| 26 | 0,2 | 2,49 | 133 |
| 27 | 0,3 | 4,95 | 64 |
| 28 | 0,4 | 7,42 | 40 |
| 29 | 0,5 | 4,37 | 73 |
| 30 | 0,7 | 9,31 | 31 |
| 31 | 0,9 | 12,31 | 22 |

Tableau 4 (Suite)

| EXEMPLE | Ro | % F | $\overline{DPn}$ |
|---|---|---|---|
| 32 | 1,1 | 11,50 | 24 |
| 33 | 2 | 13,35 | 20 |
| 34 | 4 | 24,52 | 8 |

Exemple 35

Dans un réacteur contenant un mélange de 50 g de $C_6F_{13}I$, 30 ml d'isopropanol et 0,2 g d'azobisisobutyronitrile, chauffé à reflux, on ajoute en 5 heures une solution contenant 50 g d'acrylamide, 200 ml d'isopropanol et 1,4 g d'azobisisobutyronitrile.

En fin d'addition on maintient encore 1 heure à reflux, puis on refroidit à température ambiante et on filtre le milieu réactionnel. Après lavage du solide avec trois fois 20 ml d'isopropanol et séchage à l'étuve à 70 °C, on obtient 60 g de solide pulvérulent jaunâtre dont la teneur en fluor est égale à 11,3 % ce qui correspond à un $\overline{DPn}$ de 24,5.

La tension superficielle d'une solution aqueuse contenant 1 000 ppm de ce produit est égale à 16 mN/m à 25 °C et celle d'une solution à 100 ppm égale à 26,6 mN/m.

Exemple 36

En engageant 61,2 g de $C_8F_{17}I$ dans les mêmes conditions qu'à l'exemple 35 et avec les mêmes quantités des autres réactifs, on a obtenu 64 g de télomère à 18,2 % de fluor correspondant à un $\overline{DPn}$ de 17.

La tension superficielle d'une solution aqueuse contenant 100 ppm de ce télomère est égale à 17,8 mN/m à 25 °C.

Exemple 37

Dans un réacteur, on introduit 140 ml d'isopropanol et 264,5 g d'un mélange de $R_FI$ ayant la composition pondérale suivante :

| | |
|---|---|
| $C_6F_{13}I$ | 50,9 % |
| $C_8F_{17}I$ | 25,7 % |
| $C_{10}F_{21}I$ | 13 % |
| $C_{12}F_{25}I$ | 6,3 % |
| $C_{14}F_{29}I$ | 2,6 % |
| $C_{16}F_{33}I$ | 0,9 % |
| $C_{18}F_{37}I$ | 0,1 % |

le poids moléculaire moyen étant égal à 513.

Le mélange de $R_FI$ et d'isopropanol étant chauffé à reflux, on introduit en 5 heures, sous agitation, une solution de 230 g d'acrylamide, 920 ml d'isopropanol et 6,4 g d'azobisisobutyronitrile.

En fin d'addition on rajoute encore 2 g d'azobisisobutyronitrile et on poursuit le chauffage à reflux pendant 2 heures. Le milieu réactionnel est ensuite refroidi à température ambiante et filtré. Après lavage du solide avec deux fois 150 ml d'isopropanol et séchage sous vide, on obtient 304 g de produit pulvérulent jaunâtre contenant 17,6 % de fluor, ce qui correspond à un $\overline{DPn}$ de 16,5.

Ce produit est soluble dans l'eau et dans les milieux hydro-alcooliques et permet d'abaisser de façon notable la tension superficielle de l'eau.

Tension superficielle des solutions aqueuses à 25 °C contenant :

1 000 ppm de produit fluoré : 18,9 mN/m
100 ppm de produit fluoré : 21,6 mN/m

Exemple 38

Dans les mêmes conditions qu'à l'exemple 37 mais en utilisant comme solvant la méthyléthylcétone, on a obtenu 305 g de télomère sec, contenant 16 % de fluor ce qui correspond à un $\overline{DPn}$ de 19.

Tension superficielle des solutions aqueuses à

1 000 ppm : 19,4 mN/m à 25 °C
100 ppm : 21,6 mN/m à 25 °C

Exemple 39

On introduit dans un réacteur 57 g d'un mélange $R_FI$ de composition identique à celui utilisé pour l'exemple 37, 160 ml d'isopropanol et 0,3 g d'azobisisobutyronitrile.

Le mélange est chauffé à reflux, puis on ajoute toutes les heures pendant neuf heures 5 g d'acrylamide solide et 0,14 g d'azobisisobutyronitrile de façon à introduire au total 50 g d'acrylamide et 1,4 g d'azobisisobutyronitrile. Après la dernière introduction, on maintient encore pendant une demi-heure à reflux, puis on rajoute 0,3 g d'azobisisobutyronitrile et on maintient encore une heure à reflux. Après refroidissement, filtration, lavage avec deux fois 30 ml d'isopropanol et séchage, on obtient 69 g de télomère solide contenant 14,6 % de fluor ce qui correspond à un $\overline{DPn}$ de 21,5.

Tension superficielle des solutions aqueuses à

1 000 ppm : 19,7 mN/m à 25 °C
100 ppm : 21,1 mN/m à 25 °C

Exemple 40 : Télomérisation de l'acide méthacrylique avec $C_6F_{13}CH_2CH_2I$

En opérant comme aux exemples 10 à 16, mais en remplaçant l'acrylamide par l'acide méthacrylique avec un rapport molaire $C_6F_{13}CH_2CH_2I$/acide méthacrylique égal à 4, on a obtenu un télomère contenant 3,84 % de fluor, ce qui correspond à un $\overline{DPn}$ d'environ 69.

Exemple 41

On introduit dans un réacteur 125 g de $C_6F_{13}I$, 125 ml d'isopropanol et 1,5 g d'azobisisobutyronitrile. Le mélange est chauffé à reflux, puis on ajoute en 5 heures un mélange de 250 g d'acrylamide, 750 ml d'isopropanol et 7 g d'azobisisobutyronitrile. En fin d'addition, on maintient une heure à reflux, puis on ajoute 1,25 g d'azobisisobutyronitrile et on maintient encore à reflux pendant deux heures. On introduit ensuite 400 g d'eau dans le réacteur et on élimine par distillation la plus grande partie de l'isopropanol. On a ainsi obtenu 663 g de distillat contenant 20 g de $C_6F_{13}H$, mais ne contenant pas de $C_6F_{13}I$. Le taux de transformation du $C_6F_{13}I$ a été quantitatif.

Le résidu de distillation (769 g) a fourni, après séchage à l'étuve à 100 °C, 360 g de produit sec contenant 14,3 % de fluor, ce qui correspond à un télomère ayant un $\overline{DPn}$ voisin de 18.

Exemple 42

On introduit dans un réacteur 62,5 g de $C_6F_{13}I$, 48,5 g d'acétonitrile et 1,1 g d'azobisisobutyronitrile, puis on chauffe ce mélange jusqu'à reflux.

Sous agitation et à reflux, on ajoute alors en 3 heures et demie une solution de 125 g d'acrylamide dans 292,5 g d'acétonitrile, ainsi que 1,1 g d'azobisisobutyronitrile toutes les 70 minutes.

Après addition complète de l'acrylamide, on chauffe une heure et demie à reflux, puis on ajoute 0,65 g d'azobisisobutyronitrile et chauffe encore à reflux pendant une heure. On introduit ensuite 200 g d'eau dans le mélange réactionnel qui se sépare en deux phases après refroidissement. La phase supérieure est constituée d'acétonitrile. La phase inférieure (352 g) est une solution de télomère à 45,8 % d'extrait sec et contenant 6,44 % de fluor, ce qui correspond à un $\overline{DPn}$ de 18,5.

Exemple 43

Dans un réacteur, on charge 30 ml d'acétone, 0,2 g d'azobisisobutyronitrile et 57,5 g d'un mélange de $R_FI$ de composition pondérale suivante :

| | |
|---|---|
| $C_6F_{13}I$ | 55,8 % |
| $C_8F_{17}I$ | 26,9 % |
| $C_{10}F_{21}I$ | 11,0 % |
| $C_{12}F_{25}I$ | 4,0 % |
| $C_{14}F_{29}I$ | 1,4 % |
| $C_{16}F_{33}I$ | 0,5 % |
| $C_{18}F_{37}I$ | 0,2 % |

En chauffant à reflux et sous agitation, on ajoute en 5 heures une solution de 50 g d'acrylamide et 1,4 g d'azobisisobutyronitrile dans 200 ml d'acétone. L'addition terminée, on rajoute 0,2 g d'azobisisobutyronitrile et chauffe pendant encore une heure à reflux.

Après refroidissement à température ambiante, on filtre le solide, le lave avec trois fois 20 ml d'acétone et le sèche à l'étuve à 70 °C. On obtient ainsi 66,7 g de produit contenant 14,6 % de fluor, ce qui correspond à un $\overline{DPn}$ de 21,5.

Exemple 44

On opère comme à l'exemple précédent, mais en remplaçant l'acétone par de l'éthanol. On obtient 63 g de solide à 17,7 % de fluor, ce qui correspond à un $\overline{DPn}$ de 16,5.

Exemple 45

On place dans un réacteur 125 ml de méthyl-2 propanol-2, 2,12 g d'azobisisobutyronitrile et 115 g d'un mélange de $R_FI$ ayant la même composition qu'à l'exemple 43.

Sous agitation et léger reflux, on introduit en trois heures et demie une solution de 250 g d'acrylamide dans 750 ml de méthyl-2 propanol-2, ainsi que 2,12 g d'azobisisobutyronitrile toutes les 70 minutes.

Après addition complète de l'acrylamide, on chauffe encore pendant une heure et demie à reflux, puis rajoute 1,25 g d'azobisisobutyronitrile et prolonge le reflux pendant une heure.

On distille ensuite 261 g de méthyl-2 propanol-2, puis on ajoute 400 g d'eau et distille ensuite 350 g d'azéotrope eau/méthyl-2 propanol-2. On obtient ainsi 805,5 g d'une solution de télomères à 44,6 % d'extrait sec.

Cette solution titre 7,55 % de fluor, ce qui correspond à un $\overline{DPn}$ voisin de 17 et à un taux de conversion des $R_FI$ de 91,2 %.

L'analyse chromatographique en phase gazeuse des distillats révèle uniquement la présence des $R_FI$, qui sont recyclables, et l'absence de $R_FH$.

Utilisation des oligomères séquences comme additifs aux émulseurs protéiniques

L'influence de la présence de faibles quantités de tensioactifs fluorés selon l'invention dans les émulseurs protéiniques a été vérifiée en examinant les propriétés suivantes de ces émulseurs :

Taux d'expansion
Pouvoir moussant
Temps de décantation
Fluidité de la mousse
Contamination de la mousse par les hydrocarbures.

Les émulseurs protéiniques commerciaux existent en général sous forme de concentrés à 3 % ou 6 % qui sont dilués au moment de leur emploi avec de l'eau (eau douce, eau de mer) à raison de 3 ou 6 parties de concentré pour 100 parties de produit final. Tous les essais ont été réalisés avec une solution diluée, obtenue par dilution de 6 parties d'un concentré protéinique à 6 % avec de l'eau douce.

L'influence de la présence des tensioactifs fluorés a été vérifiée en incorporant dans une telle solution protéinique diluée une certaine quantité de tensioactif fluoré. En vue de pouvoir comparer valablement les différents tensioactifs fluorés, les essais ont été réalisés en introduisant une quantité de tensioactif fluoré correspondant toujours à la même quantité de fluor, à savoir 40 mg de fluor par litre de solution diluée. Cette quantité de 40 mg de fluor par litre correspond par exemple à 0,02 % en poids d'un tensioactif fluoré à 20 % de fluor.

Différentes sortes de concentrés protéiniques ont été utilisées car les propriétés d'un émulseur protéinique donné dépendent à la fois de la nature des protéines utilisées, de la méthode d'hydrolyse et des différents ingrédients éventuellement ajoutés.

Les caractéristiques de la mousse ont été déterminées selon un certain nombre de tests simples qui permettent de bien vérifier l'influence de l'addition du tensioactif fluoré.

a) Taux d'expansion et temps de décantation

On transforme 100 ml de solution diluée en mousse à l'aide d'un batteur ménager (Moulinex type 116.2.02) muni de deux fouets. Les 100 ml de solution moussante sont introduits dans le bol et le batteur est mis en marche pendant 2 minutes et 15 secondes. Au bout de ce temps, le liquide est en général entièrement transformé en mousse et cette mousse est versée le plus rapidement possible dans une éprouvette graduée avec un fond conique permettant de mesurer son volume et son temps de décantation. On définit le taux d'expansion (TE) par le rapport :

$$TE = V/v$$

où

V désigne le volume de mousse obtenue et

v le volume de solution aqueuse effectivement transformé en mousse, c'est-à-dire 100 ml si tout le liquide a bien été transformé entièrement en mousse.

Le temps de décantation (TD) est le temps nécessaire pour recueillir dans l'éprouvette graduée un

volume de liquide correspondant au quart du volume de solution moussante transformée en mousse c'est-à-dire en principe 25 ml.

b) Pouvoir moussant (PM)

Le pouvoir moussant est le volume de mousse obtenu en versant à l'aide d'un entonnoir 500 ml de solution moussante dans 100 ml de la même solution moussante placés au fond d'une éprouvette graduée de 2 litres. Le diamètre de l'entonnoir est de 160 mm et la base de cet entonnoir (diamètre intérieur : 13 mm) se trouve à 450 mm au-dessus de la surface des 100 ml de solution. Le volume de mousse est mesuré 30 secondes après l'introduction des 500 ml de solution moussante dans l'éprouvette.

c) Fluidité de la mousse

La fluidité de la mousse est déterminée par la vitesse d'écoulement (VE) de la mousse sur un plan incliné. A cet effet, on utilise une boîte en PVC dont le fond est percé de trous et qui est inclinée de 10° par rapport à l'horizontale. Cette boîte dont les dimensions sont :

| | |
|---|---|
| hauteur | 10 cm |
| largeur | 21 cm |
| longueur | 35 cm |

comporte un compartiment (10 × 21 × 10,5 cm) permettant de recevoir 2 litres de mousse et séparé du reste de la boîte par un volet mobile. Le compartiment est rempli avec 2 litres de mousse obtenue à l'aide du batteur comme décrit dans le paragraphe a) concernant l'expansion et la décantation de la mousse, puis on libère cette mousse en enlevant le volet mobile. On note ensuite le temps au bout duquel la mousse a recouvert totalement le plan incliné, c'est-à-dire le fond de la boîte. Plus ce temps est petit, plus la mousse est mobile. Lorsque la mousse n'a pas recouvert tout le plan incliné au bout de 60 secondes, on note pour ce temps le pourcentage de la surface du plan incliné recouvert de mousse.

d) Contamination de la mousse par les hydrocarbures

Le pouvoir moussant de certains émulseurs protéiniques est sévèrement perturbé par la présence d'hydrocarbures. Cette tolérance vis-à-vis des hydrocarbures peut être caractérisée par la mesure de la mousse formée en présence d'hydrocarbures, au moyen du test suivant :

On agite pendant une heure avec un agitateur rotatif un mélange de 50 ml de cyclohexane et de 50 ml de la solution moussante à tester, ce mélange étant contenu dans un flacon en verre d'un litre de capacité. Après l'arrêt de l'agitation on mesure la hauteur de mousse (HM) formée dans le flacon. Plus cette hauteur de mousse est importante, meilleure est la tolérance de la mousse à la contamination par les hydrocarbures.

Le tableau 5 suivant rassemble les résultats obtenus dans les tests décrits ci-dessus à partir d'un même concentré protéinique sodique dilué à la dose d'emploi avec de l'eau douce, puis additionné d'une quantité de tensioactif fluoré correspondant à 40 mg de fluor par litre de solution diluée. A titre comparatif, on a réalisé un essai témoin (sans tensioactifs) et des essais utilisant des tensioactifs fluorés commerciaux, non conformes à la présente invention, à savoir :

Forafac 1110 : tensioactif fluoré non ionique
Forafac 1119 : sel de potassium d'un acide carboxylique à chaîne perfluorée
Forafac 1157 : tensioactif fluoré amphotère.

L'examen des résultats permet de constater que le Forafac 1110 agit comme agent anti-mousse. Les deux autres produits commerciaux (Forafac 1119 et Forafac 1157) ont un meilleur comportement ; la vitesse d'écoulement (VE) est excellente, mais aux dépens du pouvoir moussant. Par ailleurs, la tolérance aux hydrocarbures (HM) n'est bonne dans aucun des cas.

Tous les produits selon l'invention permettent d'améliorer de façon notable les performances des mousses, en particulier le temps de décantation, la vitesse d'écoulement et la tolérance à la contamination par les hydrocarbures.

(Voir Tableau 5 p. 11)

En vue de vérifier que les produits selon l'invention permettent d'améliorer les hydrolysats de protéine de différentes provenances, on a testé le télomère de l'exemple 37 avec deux autres émulseurs protéiniques. L'émulseur I est un hydrolysat de protéine calcique et l'émulseur II est un hydrolysat sodique comme celui utilisé dans les tests précédents, mais avec des propriétés différentes. Les résultats indiqués dans le tableau 6 ci-dessous permettent de constater que les deux émulseurs sont nettement améliorés par l'ajout d'un tensioactif du type télomère biséquencé selon l'invention.

Tableau 5

| TENSIO-ACTIF FLUORE | PROPRIETES TE | TD (secondes) | PM (ml) | VE (secondes) | HM (cm) |
|---|---|---|---|---|---|
| Néant (témoin) | 7,5 | 190 | 380 | - (35 % en 60 sec) | 1,5 |
| **Produits de commerce :** | | | | | |
| Forafac 1110 | 0,5 | - | 85 | - | 1,5 |
| Forafac 1119 | 7 | 170 | 340 | 14 | 1 |
| Forafac 1157 | 9 | 300 | 300 | 11 | 3 |
| **Télomère selon l'exemple :** 17 | 8,5 | 210 | 380 | 6 | 5 |
| 35 | 9 | 295 | 390 | 6 | 4 |
| 36 | 9 | 245 | 400 | 5 | 6 |
| 37 | 9 | 300 | 600 | 8 | 4,5 |
| 38 | 8,5 | 300 | 360 | 4 | 6,5 |
| 39 | 8,5 | 330 | 300 | 9 | 5 |

Tableau 6

| PROTEINE | I | | II | |
|---|---|---|---|---|
| Tensioactif fluoré | Néant | ex. 37 | Néant | Ex.37 |
| Propriétés : | | | | |
| TE | 7,5 | 8,5 | 7,5 | 9 |
| TD (secondes) | 225 | 385 | 130 | 230 |
| PM (ml) | 440 | 500 | 200 | 300 |
| VE (secondes) | 20 % en 60 sec. | 25 | 4 | 4 |
| HM (cm) | 1 | 4 | 0,5 | 2 |

**Revendications** (pour les Etats contractants : BE, CH, DE, FR, GB, IT, LI, LU, NL, SE)

1. Télomères fluorés qui peuvent être représentés par la formule générale :

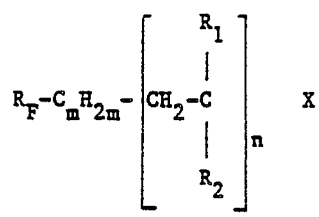

(I)

dans laquelle $R_F$ représente un radical poly- ou perfluoré, X représente un atome d'iode, de chlore ou de brome, m est égal à 0, 1 ou 2, n est un nombre allant de 5 à 1 000, $R_1$ représente un atome d'hydrogène ou un radical méthyle, $R_2$ représente un groupe —COOH ou $—CONR_3R_4$, les symboles $R_3$ et $R_4$, identiques ou différents, représentant chacun un atome d'hydrogène ou un radical alkyle ou hydroxyalkyle.

2. Télomères fluorés selon la revendication 1, dans lesquels $R_F$ représente un radical perfluoré $C_xF_{2x+1}—$, linéaire ou ramifié, x étant un nombre allant de 1 à 20, de préférence compris entre 4 et 16.

3. Télomères selon la revendication 1 ou 2, dans lesquels X est un atome d'iode.

4. Télomères fluorés selon l'une des revendications 1 à 3, dans lesquels $R_1$ est un atome d'hydrogène et $R_2$ un groupe $—CONH_2$.

5. Télomères fluorés selon l'une des revendications 1 à 4, dans lesquels m est égal à 2.

6. Télomères fluorés selon l'une des revendications 1 à 4, dans lesquels m est égal à zéro.

7. Procédé de préparation de télomères fluorés, caractérisé en ce que l'on fait réagir par voie radicalaire un monomère éthylénique de formule :

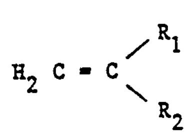

(II)

avec un télogène de formule :

$$R_F—C_mH_{2m}—X \quad (III)$$

les symboles $R_1$, $R_2$, $R_F$, m et X ayant les mêmes significations que dans la revendication 1.

8. Procédé selon la revendication 7, dans lequel on effectue la réaction dans un solvant inerte à une température comprise entre 30 et 100 °C.

9. Procédé selon la revendication 7 ou 8, dans lequel $R_F$ représente un radical perfluoré $C_xF_{2x+1}$— tel que défini dans la revendication 2.

10. Procédé selon l'une des revendications 7 à 9, dans lequel X est un atome d'iode.

11. Procédé selon l'une des revendications 7 à 10, dans lequel le monomère éthylénique est l'acrylamide.

12. Procédé selon l'une des revendications 7 à 11, dans lequel m est égal à 2.

13. Procédé selon l'une des revendications 7 à 11, dans lequel m est égal à zéro.

14. Utilisation des télomères fluorés selon l'une des revendications 1 à 6 comme agents tensioactifs en milieu aqueux.

15. Utilisation des télomères fluorés selon l'une des revendications 1 à 6 comme additifs aux émulseurs protéiniques anti-incendie.

16. Emulseurs protéiniques anti-incendie contenant un télomère fluoré selon l'une des revendications 1 à 6.

**Revendications** (pour l'Etat contractant AT)

1. Procédé de préparation de télomères fluorés qui peuvent être représentés par la formule générale :

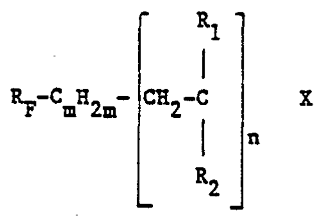

(I)

dans laquelle $R_F$ représente un radical poly- ou perfluoré, X représente un atome d'iode, de chlore ou de brome, m est égal à 0, 1 ou 2, n est un nombre allant de 5 à 1 000, $R_1$ représente un atome d'hydrogène ou un radical méthyle, $R_2$ représente un groupe —COOH ou —$CONR_3R_4$, les symboles $R_3$ et $R_4$, identiques ou différents, représentant chacun un atome d'hydrogène ou un radical alkyle ou hydroxyalkyle, caractérisé en ce que l'on fait réagir par voie radicalaire un monomère éthylénique de formule :

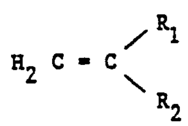

(II)

avec un télogène de formule :

$$R_F—C_mH_{2m}—X \quad (III)$$

les symboles $R_1$, $R_2$, $R_F$, m et X ayant les mêmes significations que ci-dessus.

2. Procédé selon la revendication 1, dans lequel $R_F$ représente un radical perfluoré $C_xF_{2x+1}$—, linéaire ou ramifié, x étant un nombre allant de 1 à 20, de préférence compris entre 4 et 16.

3. Procédé selon la revendication 1 ou 2, dans lequel X est un atome d'iode.

4. Procédé selon l'une des revendications 1 à 3, dans lequel $R_1$ est un atome d'hydrogène et $R_2$ un groupe —$CONH_2$.

5. Procédé selon l'une des revendications 1 à 4, dans lequel m est égal à 2.

6. Procédé selon l'une des revendications 1 à 4, dans lequel m est égal à zéro.

7. Procédé selon l'une des revendications 1 à 6, dans lequel on effectue la réaction dans un solvant inerte à une température comprise entre 30 et 100 °C.

8. Utilisation des télomères fluorés tels que définis dans l'une des revendications 1 à 6 comme agents tensioactifs en milieu aqueux.

9. Utilisation des télomères fluorés tels que définis dans l'une des revendications 1 à 6 comme additifs aux émulseurs protéiniques anti-incendie.

10. Emulseurs protéiniques anti-incendie contenant un télomère fluoré, tel que défini dans l'une des revendications 1 à 6.

**Claims** (for the Contracting States : BE, CH, DE, FR, GB, IT, LI, LU, NL, SE)

1. Fluorinated telomers which may be represented by the general formula :

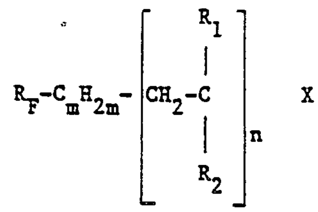

(I)

in which $R_F$ denotes a poly- or perfluorinated radical, X denotes an iodine, chlorine or bromine atom, m equals 0, 1 or 2, n is a number ranging from 5 to 1 000, $R_1$ denotes a hydrogen atom or a methyl radical, and $R_2$ denotes a —COOH or $—CONR_3R_4$ group, the symbols $R_3$ and $R_4$, which may be identical or different, each denoting a hydrogen atom or an alkyl or hydroxyalkyl radical.

2. Fluorinated telomers according to Claim 1, in which $R_F$ denotes a linear or branched perfluorinated radical $C_xF_{2x+1}—$, x being a number ranging from 1 to 20, preferably between 4 and 16.

3. Telomers according to Claim 1 or 2, in which X is an iodine atom.

4. Fluorinated telomers according to one of Claims 1 to 3, in which $R_1$ is a hydrogren atom and $R_2$ a $—CONH_2$ group.

5. Fluorinated telomers according to one of Claims 1 to 4, in which m equals 2.

6. Fluorinated telomers according to one of Claims 1 to 4, in which m equals zero.

7. Process for preparing fluorinated telomers, characterised in that an ethylenic monomer of formula :

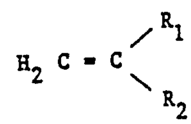

(II)

is reacted by a free-radical reaction with a telogen of formula :

$$R_F—C_mH_{2m}—X \qquad (III)$$

the symbols $R_1$, $R_2$, $R_F$, m and X having the same meanings as in Claim 1.

8. Process according to Claim 7, in which the reaction is performed in an inert solvent at a temperature between 30 and 100 °C.

9. Process according to Claim 7 or 8, in which $R_F$ denotes a perfluorinated radical $C_xF_{2x+1}—$ as defined in Claim 2.

10. Process according to one of Claims 7 to 9, in which X is an iodine atom.

11. Process according to one of Claims 7 to 10, in which the ethylenic monomer is acrylamide.

12. Process according to one of Claims 7 to 11, in which m equals 2.

13. Process according to one of Claims 7 to 11, in which m equals zero.

14. Use of the fluorinated telomers according to one of Claims 1 to 6 as surfactants in an aqueous medium.

15. Use of the fluorinated telomers according to one of Claims 1 to 6 as additives to fire-fighting protein-based emulsifiers.

16. Fire-fighting protein-based emulsifiers containing a fluorinated telomer according to one of Claims 1 to 6.

**Claims** (for the Contracting State AT)

1. Process for preparing fluorinated telomers which may be represented by the general formula :

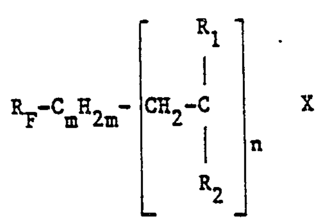

(I)

in which $R_F$ denotes a poly- or perfluorinated radical, X denotes an iodine, chlorine or bromine atom, m equals 0, 1 or 2, n is a number ranging from 5 to 1 000, $R_1$ denotes a hydrogen atom or a methyl radical,

and $R_2$ denotes a —COOH or —$CONR_3R_4$ group, the symbols $R_3$ and $R_4$, which may be identical or different, each denoting a hydrogen atom or an alkyl or hydroxyalkyl radical, characterised by reacting an ethylenic monomer of formula :

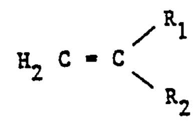

(II)

by a free-radical reaction with a telogen of formula :

$$R_F—C_mH_{2m}—X \quad \text{(III)}$$

the symbols $R_1$, $R_2$, $R_F$, m and X being as defined above.

2. Process according to claim 1, in which $R_F$ denotes a linear or branched perfluorinated radical $C_xF_{2x+1}$—, x being an integer from 1 to 20, preferably comprised between 4 and 16.

3. Process according to claim 1 or 2, in which X is an iodine atom.

4. Process according to one of claims 1 to 3, in $R_1$ is a hydrogen atom and $R_2$ a —$CONH_2$ group.

5. Process according to one of claims 1 to 4, in which m equals 2.

6. Process according to one of claims 1 to 4, in which m equals zero.

7. Process according to one of claims 1 to 6, in which the reaction is performed in an inert solvent at a temperature comprised between 30 and 100 °C.

8. Use of the fluorinated telomers such as defined in any one of claims 1 to 6 as surfactants in an aqueous medium.

9. Use of the fluorinated telomers such as defined in any one of claims 1 to 6 as additives to firefighting protein-based emulsifiers.

10. Fire-fighting protein-based emulsifiers containing a fluorinated telomer such as defined in any one of claims 1 to 6.

**Patentansprüche** (für die Vertragsstaaten : BE, CH, DE, FR, GB, IT, LI, LU, NL, SE)

1. Fluorierte Telomere gemäß der nachstehenden allgemeinen Formel :

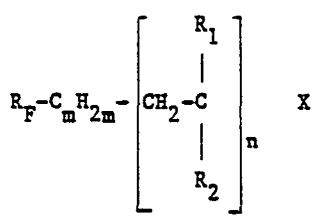

(I)

in der $R_F$ einen poly- oder perfluorierten Rest darstellt, X ein Jod, Chlor- oder Bromatom darstellt, m gleich 0, 1 oder 2 ist, n eine Zahl zwischen 5 und 1 000 ist, $R_1$ ein Wasserstoffatom oder einen Methylrest darstellt, $R_2$ eine —COOH oder —$CONR_3R_4$-Gruppe darstellt, wobei die Symbole $R_3$ und $R_4$ identisch oder unterschiedlich sind und jeweils ein Wasserstoffatom oder einen Alkylrest oder einen Hydroxyalkylrest darstellen.

2. Fluorierte Telomere nach Anspruch 1, bei denen $R_F$ einen linearen oder verzweigten perfluorierten Rest der Formel $C_xF_{2x+1}$— darstellt, wobei x eine Zahl zwischen 1 und 20, vorzugsweise zwischen 4 und 16 ist.

3. Telomere nach Anspruch 1 oder 2, bei denen X ein Jodatom ist.

4. Fluorierte Telomere nach einem der Ansprüche 1 bis 3, in denen $R_1$ ein Wasserstoffatom und $R_2$ eine —$CONH_2$-Gruppe darstellt.

5. Fluorierte Telomere nach einem der Ansprüche 1 bis 4, bei denen m gleich 2 ist.

6. Fluorierte Telomere gemäß einem der Ansprüche 1 bis 4, bei denen m gleich 0 ist.

7. Verfahren zur Herstellung fluorierter Telomere, dadurch gekennzeichnet, daß man im Wege radikalischer Reaktion ein ethylenisches Monomer der nachfolgenden Formel :

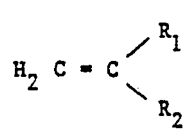

(II)

mit einem Telogen der Formel :

$$R_F\text{—}C_mH_{2m}\text{—}X \qquad \text{(III)}$$

wobei die Symbole $R_1$, $R_2$, $R_F$, m und X die gleiche Bedeutung haben wie in Anspruch 1, reagieren läßt.

8. Verfahren nach Anspruch 7, bei dem man die Reaktion in einem inerten Solvens bei einer Temperatur zwischen 30 und 100 °C durchführt.

9. Verfahren nach Anspruch 7 oder 8, bei dem $R_F$ einen perfluorierten Rest $C_xF_{2x+1}$— gemäß der Definition von Anspruch 2 darstellt.

10. Verfahren nach einem der Ansprüche 7 bis 9, bei dem X ein Jodatom ist.

11. Verfahren nach einem der Ansprüche 7 bis 10, bei dem das ethylenische Monomer Acrylamid ist.

12. Verfahren nach einem der Ansprüche 7 bis 11, bei dem m gleich 2 ist.

13. Verfahren nach einem der Ansprüche 7 bis 11, bei dem m gleich 0 ist.

14. Verwendung der fluorierten Telomeren gemäß einem der Ansprüche 1 bis 6 als im wässrigen Milieu oberflächenaktive Agentien.

15. Verwendung der fluorierten Telomeren gemäß einem der Ansprüche 1 bis 6 als Zusatz in proteinischen feuerhemmenden Emulgatoren.

16. Feuerhemmende proteinische Emulgatoren enthaltend ein fluoriertes Telomer gemäß einem der Ansprüche 1 bis 6.

**Patentansprüche** (für den Vertragsstaat AT)

1. Verfahren zur Herstellung fluorierter Telomere gemäß der nachfolgenden allgemeinen Formel :

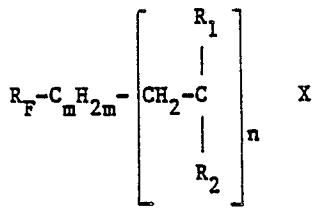

(I)

in der $R_F$ einen poly- oder perfluorierten Rest darstellt, X ein Jodatom, Chloratom oder Bromatom darstellt, m gleich 0, 1 oder 2 ist, n eine Zahl zwischen 5 und 1 000 ist, $R_1$ ein Wasserstoffatom oder einen Methylrest darstellt, $R_2$ eine —COOH oder —$CONR_3R_4$-Gruppe darstellt, wobei die Reste $R_3$ und $R_4$ identisch oder unterschiedlich sind und jeweils ein Wasserstoffatom oder einen Alkylrest oder Hydroxyalkylrest darstellen, dadurch gekennzeichnet, daß man im wege radikalischer Reaktion ein ethylenisches Monomer der Formel :

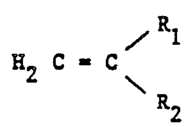

(II)

mit einem Telogen der Formel :

$$R_F\text{—}C_mH_{2m}\text{—}X \qquad \text{(III)}$$

reagieren läßt, wobei die Symbole, $R_1$, $R_2$, $R_F$, m und X die gleiche oben angeführte Bedeutung haben.

2. Verfahren nach Anspruch 1, bei dem $R_F$ einen linearen oder verzweigten perfluorierten Rest $C_xF_{2x+1}$— darstellt, wobei x eine Zahl zwischen 1 und 20, vorzugsweise zwischen 4 und 16 ist.

3. Verfahren nach Anspruch 1 oder 2, bei dem X ein Jodatom ist.

4. Verfahren nach einem der Ansprüche 1 bis 3, in dem $R_1$ ein Wasserstoffatom und $R_2$ eine —$CONH_2$-Gruppe ist.

5. Verfahren nach einem der Ansprüche 1 bis 4, bei dem m gleich 2 ist.

6. Verfahren nach einem der Ansprüche 1 bis 4, bei dem m gleich 0 ist.

7. Verfahren nach einem der Ansprüche 1 bis 6, bei dem man die Reaktion in einem inerten Solvenz bei einer Temperatur zwischen 30 und 100 °C durchführt.

8. Verwendung fluorierter Telomere gemäß der Definition eines der Ansprüche 1 bis 6, als in wässrigem Medium oberflächenaktives Agens.

9. Verwendung fluorierter Telomeren gemäß der Definition eines der Ansprüche 1 bis 6 als Zusatz zu feuerhemmenden proteinischen Emulgatoren.

10. Feuerhemmende proteinische Emulgatoren enthaltend ein fluoriertes Telomer gemäß der Definition eines der Ansprüche 1 bis 6.